# EUROPÄISCHE PATENTANMELDUNG

(11) **EP 2 374 784 A1**
(43) Veröffentlichungstag der Anmeldung: **12.10.2011**
(21) Anmeldenummer: 10159247.5
(22) Anmeldetag: 07.04.2010
(51) Int. Cl.: C07C 37/82, C07C 39/16

(54) **Reinigung von Tris-hydroxyaryl-Verbindungen**

(71) Anmelder: Bayer MaterialScience AG, 51368 Leverkusen (DE)
(72) Erfinder: Münnich, Christian, Dr., 51377 Leverkusen (DE); Konrad, Stephan, Dr., 41541 Dormagen (DE); Köhler, Karl-Heinz, Dr., 52078 Aachen-Brand (DE)

(57) **Zusammenfassung**

Gegenstand der Erfindung sind Tris-hydroxyaryl-Verbindungen, vorzugsweise 1,1,1-Tris(4-hydroxyphenyl)ethan (THPE), mit einem Gehalt an Metall-Ionen, insbesondere an Natrium-Ionen von weniger als 5 ppm, vorzugsweise von weniger als 1 ppm. Weiterer Gegenstand der Erfindung ist ein Verfahren zur Herstellung von Tris-hydroxyaryl-Verbindungen, vorzugsweise 1,1,1 -Tris(4-hydroxyphenyl)ethan (THPE), mit einem Gehalt an Metall-Ionen, insbesondere an Natrium-Ionen von weniger als 5 ppm, vorzugsweise von weniger als 1 ppm, durch Reinigung der aromatischen Hydroxy-Verbindungen an sauren Kationen-Austauscherharzen.

## Beschreibung

Gegenstand der Erfindung sind Tris-hydroxyaryl-Verbindungen, vorzugsweise 1,1,1-Tris(4-hydroxyphenyl)ethan (THPE), mit einem Gehalt an Metall-Ionen, insbesondere an Natrium-Ionen von weniger als 5 ppm, vorzugsweise von weniger als 1 ppm. Weiterer Gegenstand der Erfindung ist ein Verfahren zur Herstellung von Tris-hydroxyaryl-Verbindungen, vorzugsweise 1,1,1 -Tris(4-hydroxyphenyl)ethan (THPE), mit einem Gehalt an Metall-Ionen, insbesondere an Natrium-Ionen von weniger als 5 ppm, vorzugsweise von weniger als 1 ppm, durch Reinigung der aromatischen Hydroxy-Verbindungen an sauren Kationen-Austauscherharzen.

Tris-hydroxyaryl-Verbindungen, insbesondere THPE, sind geeignet zur Herstellung von verzweigten Polycarbonaten sowohl nach dem bekannten Phasengrenzflächenverfahren, als auch nach dem Umesterungsverfahren mit organischen Carbonaten in der Schmelze, sog. Polycarbonat-Schmelzeverfahren. Der Einsatz von THPE zur Herstellung von verzweigtem Schmelze-Polycarbonat wird zum Beispiel in der EP 1 458 786 A1 beschrieben. Das Polycarbonat-Schmelzeverfahren stellt im Gegensatz zum Phasengrenzflächenverfahren höhere Anforderungen an die Reinheit der Einsatzstoffe, da unerwünschte Nebenprodukte nicht durch eine Phasentrennung eliminiert werden können, sondern insbesondere dann, wenn sie schwer oder nicht flüchtig sind, wie z.B. Metallsalze, in der Polymerschmelze verbleiben und so zu unerwünschten Nebenreaktionen führen. Dies trifft auch für Tris-hydroxyaryl-Verbindungen zu, die als Verzweiger bei der Herstellung von verzweigtem Schmelzepolycarbonat verwendet werden und marktgängige Produkte sind. Bereits Spuren von Metallsalzen im unteren ppm-Bereich, die als Nebenprodukte oder Verunreinigungen in solchen Verzweigern enthalten sein können, haben negative Auswirkungen auf die Katalyse des Schmelzepolycarbonat-Verfahrens und führen, wie allgemein bekannt, zu einem Schmelzepolycarbonat mit einem deutlich erhöhten Anteil an Umlagerungs-Produkten, die z.B. durch sog. Fries-Verschiebung (A) entstehen. 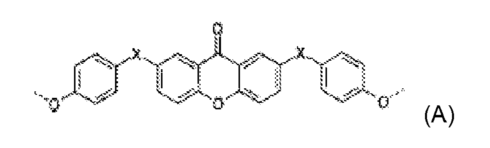 mit X= Isopropyliden-Rest.

Die Art und Menge dieser Fehlstrukturen ist abhängig von verschiedenen Verfahrensparametern, wie z. B. Temperatur, Verweilzeit und vor allem auch Art und Menge des eingesetzten Katalysators. Es ist weiterhin bekannt, dass Alkali- und Erdalkali-Metallverbindungen die Bildung von Fehlstrukturen begünstigen (siehe z.B. EP 1 369 446 A1 und EP 1 500 671 A1). Solche Fehlstrukturen sind im Schmelze-Polycarbonat unerwünscht, da sie die Eigenfarbe des Produkts und die Polymer-Schmelzefließfähigkeit nachteilig beeinflussen.

Da die marktgängigen Tris-hydroxyaryl-Verbindungen, wie z.B. THPE, teilweise Restgehalte an Metallionen wie z.B. Natrium-Ionen von mehr als 5 ppm enthalten, bestand also Bedarf an Tris-hydroxyaryl-Verbindungen, die weitgehend frei von metallischen Nebenprodukten sind, sowie einem industriell einsetzbaren Verfahren zur Aufreinigung von Tris-hydroxyaryl-Verbindungen.

Die Herstellung von Tris-hydroxyaryl-Verbindungen, insbesondere die Herstellung von THPE ist literaturbekannt und z.B. in EP 782 978 A1, EP 765 852 A1, EP 930 289 A1, EP 847 975 A1 oder EP 441 648 A1 beschrieben. So offenbart EP 765 852 A1 die Synthese von THPE mit Hilfe von Ionenaustauschern und Cokatalysatoren; EP 930 289 A1 offenbart die gleiche Synthese mit Hilfe von Mineralsäuren. Die Synthese der Tris-hydroxyaryl-Verbindungen erfolgt mit einem hohen Überschuss an einem der Edukte, insbesondere des Hydroxyaryls, um das Gleichgewicht in Richtung der Tris-hydroxyaryl-Verbindungen zu verschieben. Obwohl die Endprodukte insbesondere nach EP 765 852 A1 frei von Metall-Ionen sein sollten, findet man in den käuflichen Produkten Restgehalte an Natrium über 5 ppm. Mögliche Ursachen für diese metallischen Verunreinigungen sind Nachbehandlungen der Rohprodukte von THPE mit Natrium-Borhydrid, wie z.B. in EP 782 978 A1 beschrieben, oder die Verwendung Natrium-haltiger Co-Katalysatoren, wie z.B. Natrium-Mercaptopropansulfonat, wie sie die EP 847 975 A1 offenbart. Auch der Zusatz an Natrium-haltigen Stabilisatoren wie z.B. Natrium-Dithionit, z.B. offenbart in EP 441 648 A1, kann erhöhte Natrium-Gehalte im käuflichen THPE bewirken. Auch eine Reduzierung des Gehaltes anderer Nebenprodukte, wie z.B. von Schwefelverbindungen im THPE durch eine Nachbehandlung des THPE mit Natronlauge, siehe dazu EP 646 613 A1, kann erhöhte Natrium-Gehalte im THPE-Endprodukt bewirken.

Keines der obigen Zitate beschreibt ein Verfahren, das zu einem Tris-hydroxyaryl-Endprodukt führt, das weitgehend frei von metallischen Verunreinigungen ist und für die Herstellung von verzweigtem Polycarbonat nach dem Schmelzeverfahren geeignet ist, wobei die Bildung von unerwünschten Fehlstrukturen (Fries-Struturen, Xanthon-Strukturen) weitestgehend vermieden wird.

Es bestand daher Bedarf nach einem Verfahren mit hoher Ausbeute, durch das kommerzielle Tris-hydroxyaryl-Verbindungen weitestgehend von enthaltenen Metall-Kationen befreit werden.

Aufgabe der Erfindung war es daher, Tris-hydroxyaryl-Verbindungen, insbesondere THPE, mit Restgehalten an metallischen Ionen von weniger als 10 ppm, vorzugsweise von weniger als 5 ppm bereitzustellen insbesondere von 0,01 bis 5 ppm, die die Nachteile der oben genannten Herstellverfahren überwinden. Es war zudem Aufgabe der Erfindung, Restgehalte an Natrium-Ionen unterhalb von 5 ppm, bevorzugt weniger als 1ppm, insbesondere von 0,005 bis 1 ppm, zu erreichen.

Überraschenderweise wurde diese erfindungsgemäße Aufgabe dadurch gelöst, dass marktgängige, metallhaltige Tris-hydroxyaryl-Verbindungen unter genau definierten Bedingungen mit sauren Kationenaustauschern behandelt werden, ohne die dabei zu erwartende Rückspaltung zu den Ausgangsstoffen der Tris-hydroxyaryl-Verbindungen nennenswert zu beobachten. Insbesondere die erwartete säurekatalysierte Abspaltung von Phenol und Bildung von Nebenprodukten konnte nicht nennenswert festgestellt werden. Die so gereinigten Tris-hydroxyaryl-Verbindungen werden mit diesem erfindungsgemäßen Verfahren in Ausbeuten von mehr als 95% erhalten und weisen Gehalte an metallischen Ionen von kleiner 5 ppm, bevorzugt von kleiner 1 ppm auf. Sie bewirken beim Einsatz im Schmelzepolycarbonat-Verfahren als Verzweiger keine unerwünschten Nebenreaktionen oder Farbveränderungen.

Gegenstand der Erfindung ist daher ein Verfahren zur Behandlung von metallhaltigen Tris-hydroxyaryl-Verbindungen, das folgende Verfahrensschritte enthält:
a) Konditionieren eines Sulfonsäuregruppen enthaltenden aktiven Ionenaustauschers mit einem Lösemittel, das für die Handhabung der Tris-hydroxyaryl-Verbindungen geeignet ist (z.B. Methanol oder Phenol),
b) Herstellen einer Lösung der zu reinigenden Tris-hydroxyaryl-Verbindungen in einem Lösemittel, das für die Handhabung der Tris-hydroxyaryl-Verbindungen geeignet ist (z.B. Methanol oder Phenol)
c) kontinuierliches oder diskontinuierliches Kontaktieren der Tris-hydroxyaryl-Verbindung-haltigen Lösung aus b) mit einem konditionierten Ionenaustauscher aus a)
d) Abtrennen der Tris-hydroxyaryl-Verbindung-haltigen Lösung aus c) von dem konditionierten Ionenaustauscher,
e) gegebenenfalls wenigstens teilweises Entfernen des Lösemittels aus der in c) gereinigten Lösung der Tris-hydroxyaryl-Verbindung unter geringer Temperatur-Belastung, vorzugsweise im Vakuum,
f) optional, unter der Maßgabe, dass das Lösemittel nur teilweise oder nicht entfernt wurde, anschließendes Fällen der Tris-hydroxyaryl-Verbindung in einem geeigneten Fällungsmittel und schonendes Trocknen unter geringer Temperatur-Belastung, vorzugsweise im Vakuum.

Erfindungsgemäß zu verwendende Tris-hydroxyaryl-Verbindungen sind z.B.: Phloroglucin, 3,3-Bis(3-methyl-4-hydroxyphenyl)-2-oxo-2,3-dihydroindol, 4,6-Dimethyl-2,4,6-tris(4-hydroxyphenyl)hepten-2, 4,6-Dimethyl-2,4,6-tris(4_hydroxyphenyl)-heptan, 1,3,5-Tris-(4-hydroxyphenyl)-benzol, 1,1,1-Tris(4-hydroxyphenyl)ethan (THPE), Tris(4-hydroxyphenyl)phenylmethan, 2,2-Bis[4,4-bis(4-hydroxyphenyl)cyclohexyl]propan, 2,4-Bis-(4-hydroxyphenylisopropyl)phenol, 2,6-Bis(2-hydroxy-5'-methylbenzyl)-4-methylphenol, 2-(4-hydroxyphenyl)-2-(2,4-dihydroxyphenyl)-propan, Hexakis(4-(4-hydroxyphenylisopropyl)phenyl)orthoterephthalat, Tetrakis(4-hydroxyphenyl)methan, Tetrakis(4-(4-hydroxyphenylisopropyl)phenoxy)methan, 1,4-Bis((4',4"-dihydroxytriphenyl)methyl)benzol und Isatinbiskresol, ggf. auch Pentaerythrit.
Bevorzugtes Verzweigungsmittel ist THPE.

Erfindungsgemäß einsetzbare Ionenaustauscher sind beispielsweise Kationenaustauscher, die als Polymermatrix ein vernetztes oder teilvernetztes Polystyrol, zum Beispiel mit Divinylbenzol vernetztes Polystyrol, oder ein vernetztes oder teilvernetztes Polyacrylat, zum Beispiel mit Divinylbenzol vernetzte Polyacrylsäure, enthalten, mit Vernetzungsgraden von 1 bis 20%, vorzugsweise von 1 bis 10%, und die an die Polymermatrix chemisch gebundene Sulfonsäuregruppen in der H-Form (oder aciden Form) aufweisen. Typische Sulfonsäurekonzentrationen der sauren Kationenaustauscher liegen beispielsweise im Bereich von 3 bis 7 mol / kg Trockenmasse des Kationenaustauschers. Diese Ionenaustauscher können gegebenenfalls weitere kovalent oder ionisch gebundene Cokatalysatoren chemisch gebunden enthalten; sie sind makroporös oder gelförmig, wie es in US-A-4,191,843 und US-A-3,037,052 beschrieben ist, und weisen eine feinteilige Kugelform, Pulverform oder Membranform auf.

Der saure Ionenaustauscher kann mit einer oder mehreren Funktionen aus der Gruppe Sulfonsäure, Carbonsäure, Phosphonsäure und Perchlorsäure funktionalisiert sein.

Es können auch ein oder mehrere Ionenaustauscher gleichzeitig oder nacheinander durch Wiederholen wenigstens der Schritte (a) bis (d) verwendet werden. Dies hat den besonderen Vorteil, dass beim Verwenden mehrerer Ionenaustauscher diese auf die jeweilig zu entfernenden Ionen abgestimmt werden können.

In Schritt a) sollte die Konditionierung der erfindungsgemäß einsetzbaren Kationenaustauscher vorzugsweise mit dem gleichen Lösemittel oder Lösemittelgemisch vorgenommen werden, das auch zur Auflösung der zu reinigenden Tris-hydroxyaryl-Verbindungen verwendet wird Besonders bevorzugtes Lösemittel ist Methanol oder Phenol.

Die Konditionierung von Ionenaustauschern beinhaltet zwei Einzelschritte. In einem ersten Schritt wird der Ionenaustauscher mit Wasser gespült, um evtl. enthaltene Verunreinigungen wie Säurespuren zu entfernen (Wäsche). In einem zweiten Schritt wird der gereinigte, wasserfeuchte Ionenaustauscher mit einem geeigneten Lösemittel kontaktiert, um das enthaltene Wasser zu entfernen (Lösemittelaustausch). Je nach Mischbarkeit von Wasser und dem Lösemittel, auf das der Ionenaustauscher konditioniert werden soll, kann möglicherweise ein mehrfacher Lösemittelaustausch notwendig sein. Bevorzugt erfolgt die Konditionierung mit Methanol oder Phenol. Die Konditionierung auf andere Lösemittel kann in analoger Weise durchgeführt werden.

Bei der Konditionierung wird in einem ersten Schritt ein sulfonsaurer Ionenaustauscher, z.B. Lewatit K1221 (Lanxess AG), bei Temperaturen von 20°C bis 90°C, bevorzugt von 40°C bis 80°C, mit vollentsalztem Wasser elektrolytfrei gewaschen. Wird der Ionenaustauscher in einer Säule gewaschen, liegt der Volumenstrom im Bereich von 0,1 bis 4, bevorzugt von 0,2 bis 2, Ionenaustauscherbettvolumen pro Sunde gehalten. Der Ionenaustauscher wird so lange mit vollentsalztem Wasser elektrolytfrei gewaschen, bis die Leitfähigkeit im Ablauf der Säule bevorzugt weniger als 50 µS/cm, besonders bevorzugt weniger als 20 µS/cm, beträgt. Bei einer absatzweise durchgeführten Ionenaustauscherwäsche mit vollentsalztem Wasser wird die Wäsche so oft wiederholt, bis Leitfähigkeit in der wässrigen Phase nach einer Kontaktzeit mit dem Ionenaustauscher von 1 bis 12 h, bevorzugt von 2 bis 6 h, bevorzugt weniger als 50 µS/cm beträgt, besonders bevorzugt weniger als 20 µS/cm beträgt. Die wässrige lonenaustauschersuspension kann dabei leicht gerührt werden, um den Stofftransport zu verbessern, wobei das Rühren/Durchmischen so schonend durchgeführt werden muss, dass der Ionenaustauscher nicht beschädigt wird.

Anschließend kann der gewaschene Ionenaustauscher von der flüssigen Wasserphase z.B. durch Filtration oder Dekantieren getrennt werden.

In einem zweiten Schritt wird der elektrolytfrei gewaschene Ionenaustauscher bei Temperaturen von 0°C bis 60°C, bevorzugt von 20°C bis 50°C, so lange mit dem Lösemittel, bevorzugt Methanol oder Phenol, gespült, bis der Wassergehalt im alkoholischen Ablauf als 2 Gew.-%, bevorzugt kleiner als 0,5 Gew.-%, ist. Wird der Ionenaustauscher in einer Säule konditioniert, liegt dabei der Volumenstrom im Bereich von 0,1 bis 4, bevorzugt von 0,2 bis 2, lonenaustauscherbettvolumen pro Stunde gehalten, bis der gewünschte Wassergehalt im alkoholischen Ablauf erreicht ist.

Eine andere Ausführungsform der Konditionierung wird absatzweise durchgeführt, wobei der elektrolytfrei gewaschene Ionenaustauscher mit dem Lösemittel, bevorzugt Methanol oder Phenol, beaufschlagt wird und der Ionenaustauscher anschließend von dem flüssigen Lösemittel-Wasser-Gemisch, bevorzugt Methanol-Wasser-Gemisch oder Phenol-Wasser-Gemisch, getrennt wird. Dieser Schritt wird so oft wiederholt, bis das Lösemittel-Wasser-Gemisch 2 Gew.-% Wasser, bevorzugt weniger als 0,5 Gew.-% Wasser, enthält.

Der so auf dem Lösemittel, bevorzugt Methanol oder Phenol, konditionierte Ionenaustauscher kann für die Reinigung von Lösungen, die Tris-hydroxyaryl-Verbindungen enthalten, eingesetzt werden.

In Schritt b) erfolgt die Herstellung der Lösungen der erfindungsgemäß einzusetzenden Tris-hydroxyaryl-Verbindungen vorzugsweise durch Lösen der Tris-hydroxyaryl-Verbindungen in destilliertem Lösemittel in Konzentrationen von 10 g/l bis 500 g/l. Geeignete Lösemittel sind Alkohole, insbesondere Methanol, Phenol, Wasser oder andere protische Lösemittel, in denen die zu reinigende Tris-hydroxyaryl-Verbindung eine ausreichende Löslichkeit hat. Auch aprotische Lösemittel sind geeignet, soweit die Tris-hydroxyaryl-Verbindungen ausreichend gut darin gelöst werden und die aprotischen Lösemittel mit dem sauren Ionentauscher keine Nebenreaktionen eingehen, wie z.B. Ether- oder Esterspaltung. Geeignete aprotische Lösemittel sind z.B. Acetonitril, Dimethylsulfoxid oder Chlorbenzol. Es können auch mehrere Lösemittel im Gemisch verwendet werden.

Bevorzugt sind Lösemittel, die von der Tris-hydroxyaryl-Verbindung entfernt werden können, ohne diese thermisch besonders zu belasten.

Die Behandlung der Lösungen in Schritt c) der erfindungsgemäß einzusetzenden Tris-hydroxyaryl-Verbindungen mit den erfindungsgemäß einsetzbaren Ionenaustauschern erfolgt durch Kontaktieren der Tris-hydroxyaryl-Verbindung-haltigen Lösung mit dem Ionenaustauscher. Die Kontaktierung kann sowohl kontinuierlich als auch diskontinuierlich in geeigneten Apparaten durchgeführt werden. Geeignete Apparate für kontinuierlich oder diskontinuierlich durchgeführte Verfahren wie z.B. Säulen, Kolonnen oder gerührte Behälter sind dem Fachmann bekannt.

So kann beispielsweise die Tris-hydroxyaryl-Verbindung-haltige Lösung mit dem Ionenaustauscher in einem gerührten Behälter oder Mischern allgemeiner Art wie z.B. Taumelmischern bei Temperaturen von -50°C bis 150°C, vorzugsweise von -10°C bis 120°C, besonders bevorzugt von 10°C bis 90°C, über Verweilzeiten von 0,1 h bis 6 h, vorzugsweise von 0,25 h bis 4 h, in Kontakt gebracht werden.

Bei einer bevorzugten Ausführungsform des erfindungsgemäßen Verfahrens wird die zu reinigende Tris-hydroxyaryl-Verbindungs-haltige Lösung in Schritt c) durch Schüttungen von Ionenaustauschern z.B. in Form von sogenannten Festbetten in geeigneten temperierbaren Gefäßen, wie z.B. in Kolonnen geleitet. Dabei können Temperaturen von -50°C bis 150°C, vorzugsweise -10°C bis 120°C, besonders bevorzugt von 10°C bis 90°C, und Verweilzeiten von 0,1 h bis 6 h, vorzugsweise von 0,25 h bis 4 h, verwendet werden.

Um die erfindungsgemäßen Rest-Gehalte an ionischen Metallen in den Tris-hydroxyaryl-Verbindungen durch die Maßnahmen a) oder b) zu erhalten, kann es ggf. erforderlich sein, die Tris-hydroxyaryl-Verbindungs-haltige Lösung wiederholt mit dem aktiven Kationenaustauscher zu kontaktieren.

Der Ionenaustauscher kann mehrfach verwendet werden. Durch Kontaktieren des Ionenaustauschers mit der Tris-hydroxyaryl-Verbindung-haltigen Lösung wird der Ionenaustauscher mit Metallionen beladen, die in der Lösung enthalten sind. Dadurch sinkt die Zahl der für die Reinigung der Tris-hydroxyaryl-Verbindung-haltigen Lösung zur Verfügung stehenden aktiven Zentren, z.B. Sulfonsäure-Gruppen in der protonierten Form (H-Form). Lässt die Reinigungswirkung des Ionenaustauschers nach, kann der Ionenaustauscher getauscht oder regeneriert werden. Für die Regeneration des metallbeladenen Ionenaustauschers können die bekannten Verfahren, z.B. Spülen mit einer Protonen-haltigen Säure (Brönstedtsäure), z.B. verdünnte Salzsäure, und anschließende Neutralwäsche verwendet werden. Solche Verfahren zur Regenerierung von sauren Ionenaustauschern sind z.B. beschrieben in EP 324 080 A1, DE-A 2 727 866 oder US 4 443 635.

Das Abtrennen in Schritt d) der Tris-hydroxyaryl-Verbindung-haltigen Lösung aus c) von dem konditionierten Ionenaustauscher erfolgt durch dem Fachmann bekannte Verfahren zur Fest/flüssig-Trennung. Solche Verfahren sind z.B. Filtration oder Dekantieren. Dabei wird der feste lonentaustauscher durch ein geeignetes Feststoffrückhaltemedium, z.B. ein Filter, von der flüssigen Tris-hydroxyaryl-Verbindungs-haltigen Lösung abgetrennt.

Die so erhaltene Lösung, enthaltend wenigstens ein Lösemittel und eine Tris-hydroxyaryl-Verbindung, kann gegebenenfalls ohne weitere Schritte verwendet werden. Insbesondere wenn als Lösemittel Phenol eingesetzt wurde, so kann die Lösung als solches, gegebenenfalls nach einem weiteren Reinigungsschritt, z.B. zur Entfernung von Partikeln, verwendet werden.

Die gegebenenfalls durchzuführende wenigstens teilweise Entfernung des Lösemittels aus der am Ionenaustauscher gereinigten Lösung der Tris-hydroxyaryl-Verbindung in Schritt e) sollte zur Vermeidung von Nebenreaktionen unter möglichst geringer thermischer Belastung stattfinden; bevorzugte Temperaturen liegen im Bereich von -20°C bis 100°C, vorzugsweise von -10°C bis 80°C, besonders bevorzugt von 0°C bis 60°C. Das Abdestillieren des Lösemittels im Vakuum zur Reduzierung der thermischen Belastung ist daher bevorzugt.

Als besonders schonend hat sich in Schritt f) das Fällen der Tris-hydroxyaryl-Verbindung in einem geeigneten Fällungsmittel erwiesen. Die Fällung kann auch nach vorheriger Aufkonzentration der Tris-hydroxyaryl-Verbindung in dem Lösemittel erfolgen. Die ausgefällte Tris-hydroxyaryl-Verbindung kann durch dem Fachmann bekannte Verfahren zur Fest/flüssig-Trennung von der flüssigen Lösung abgetrennt werden. Solche Verfahren sind z.B. Filtration oder Dekantieren. Die so erhaltene feste, Lösemittel-feuchte Tris-hydroxyaryl-Verbindung wird anschließend durch Trocknung von enthaltenen Lösemittelresten befreit.

Als vorteilhaft hat sich erwiesen, die Trocknung der gereinigten und gefällten Tris-hydroxyaryl-Verbindung mit geringer thermischer Belastung der Tris-hydroxyaryl-Verbindung durchzuführen. Bevorzugte Temperaturen der Trocknung liegen im Bereich von -20°C bis 100°C, vorzugsweise von -10°C bis 80°C, besonders bevorzugt von 0°C bis 60°C. Das Trocknen der gereinigten und gefällten Tris-hydroxyaryl-Verbindung im Vakuum zur Reduzierung der thermischen Belastung ist bevorzugt.

Durch die Kombination von Aufkonzentration, anschließende Fällung und schonende Trocknung konnte eine besonders hohe Ausbeute der gereinigten Tris-hydroxyaryl-Verbindung erreicht werden.

Durch Behandlung von metallionenhaltigen Tris-hydroxyaryl-Verbindungen unter diesen erfindungsgemäßen Bedingungen werden Rückspaltungen dieser Verbindungen an den erfindungsgemäß einsetzbaren Ionenaustauschern sehr effizient vermieden, so dass die gereinigten Tris-hydroxyaryl-Verbindungen in sehr hoher Ausbeute von größer 95% und in hoher Reinheit aus dem erfindungsgemäßen Reinigungsprozess zurückgewonnen werden können.

Die so gereinigten Tris-hydroxyaryl-Verbindungen sind für die Herstellung von verzweigten Schmelzepolycarbonaten besonders geeignet, da sie die Bildung von Fries-Umlagerungs-Strukturen (Xanthon-Strukturen) als sogenannte inhärente Polymerketten-Verzweigung als Nebenreaktion bei der Schmelzepolycarbonat-Herstellung reduzieren. Es lassen sich mit diesen metallionenarmen Tris-hydroxyaryl-Verbindungen gezielt verzweigte Polycarbonate mit geringem Fries-Strukturen-Gehalt (Xanthon-Strukturen) nach dem Schmelze-Polykondensations-Verfahren herstellen. Das Verhältnis von Verzweigungen zu Xanthon-Strukturen in der Polymerkette im SPC kann durch gereinigtes, an Metallionen verarmtes THPE effizient eingestellt werden kann Auf diese Weise können Schmelzepolycarbonate mit einem Verhältnis von Verzweigerstrukturen zu Xanthon-Strukturen in der Polymerkette von deutlich größer 10, vorzugsweise von größer 15 hergestellt werden.

Die gereinigten Tris-hydroxyaryl-Verbindungen werden durch die Schmelzumesterungsreaktion geeigneter Bisphenole und Kohlensäurediarylester zu Polycarbonat in Gegenwart eines geeigneten Katalysators hergestellt. Das Polycarbonat kann auch durch die Kondensation von Carbonatoligomeren, die Hydroxy- und/oder Carbonatendgruppen enthalten, und geeigneten Kohlensäurediarylestern und Bisphenolen hergestellt werden.

Im Rahmen der vorliegenden Erfindung sind unter ppb und ppm - soweit nichts anderes angegeben - Gewichtsteile zu verstehen.

Die Metallgehalte können mittels Graphitrohr Atom Absorptionsspektrophotometrie (Graphit-AAS) oder mittels Ionenchromatographie (IC) nach dem Fachmann bekannten und in der Literatur hinreichend beschriebenen Methoden bestimmt werden.

Die nachfolgenden Beispiele mit 1,1,1-Tris(4-hydroxyphenyl)ethan (THPE) als verzweigende Tris-hydroxyaryl-Verbindung sollen die Erfindung erläutern, ohne sie auf THPE einzuschränken:

### Beispiele

### Metallgehalt

Der Metall-Gehalt der untersuchten Proben wurde mittels Graphitrohr Atom Absorptionsspektrophotometrie (Graphit-AAS) bestimmt.

### Verbindung A

Die Bestimmung der Konzentration der Verbindung A erfolgt nach alkalischer Hydrolyse des Polycarbonats und anschließender Analyse des Hydrolysates per HPLC. Die Verbindung wurde durch Kemmagnetresonanzspektroskopie charakterisiert.

### Gehalt an THPE

Die Bestimmung der Konzentration von THPE erfolgt nach alkalischer Hydrolyse des Polycarbonats und anschließender Analyse des Hydrolysates per HPLC. Die Kalibrierung erfolgt mit externem Standard.

### Reinigung von Metallionen-haltigem 1,1,1-Tris(4-hydroxyphenyl)ethan (THPE):

### Beispiel 1

In einer Glassäule wird ein sulfonsaurer Ionenaustauscher (Lewatit K1221, Lanxess AG) bei Raumtemperatur min vollentsalztem Wasser elektrolytfrei gewaschen, wobei der Volumenstrom konstant bei 0,3ml Wasser/ml Ionentauscher pro Stunde gehalten wird. Nach 24 h beträgt die Leitfähigkeit im Ablauf der Säule 7 µS/cm. Die flüssige Wasserphase wird abgelassen und der Ionenaustauscher mit Methanol beaufschlagt.

Anschließend wird der elektrolytfrei gewaschene Ionenaustauscher bei Raumtemperatur so lange mit Methanol gespült, bis der Wassergehalt im methanolischen Ablauf der Säule kleiner als 0,5 Gew.-% ist. Dabei wird der Volumenstrom konstant bei 0,3 ml Methanol/ml Ionentauscher pro Stunde gehalten. Der so auf Methanol konditionierte Ionenaustauscher wird für die Reinigung von Lösungen, die 1,1,1-Tris(4-hydroxyphenyl)ethan (THPE) enthalten, eingesetzt.

Über den so konditionierten Ionenaustauscher wird bei Raumtemperatur eine 20 Gew.-%ige methanolische THPE-Lösung gegeben. Der Ionenaustauscher wird dabei mit einem Volumenstrom von 0,3 ml THPE-Lösung pro ml Ionenaustauscher beaufschlagt. Anschließend wird der Ionenaustauscher zweimal mit Methanol gespült, wobei jeweils das Säulenvolumen des methanolfeuchten Ionenaustauschers als Spülvolumen eingesetzt wird. Der gesamte Säulenablauf wird aufgefangen und im Wasserstrahlvakuum bei 45°C eingeengt, bis eine ca. 50 Gew.-%ige Lösung von THPE in Methanol erhalten wird. Diese methanolische THPE-Lösung wird unter Rühren bei Raumtemperatur in das doppelte Volumen vollentsalztes Wasser gegeben. Der weiße Niederschlag wird abfiltriert und 48 h bei 50°C sowie 48 h bei 65°C im Wasserstrahlvakuum bis zur Gewichtskonstanz getrocknet.
Ausbeute: 96,9 %
Natriumgehalt 1,1,1-Tris(4-hydroxyphenyl)ethan vor Reinigung: 8,3 ppm
Natriumgehalt 1,1,1-Tris(4-hydroxyphenyl)ethan nach Reinigung: 610 ppb

### Beispiel 2

Der unter Beispiel 1 konditionierte Ionenaustauscher wird bei Raumtemperatur mit einer 20 Gew.-%igen methanolischen 1,1,1-Tris(4-hydroxyphenyl)ethan-(THPE)-Lösung mit einem Volumenstrom von 0,3 ml THPE-Lösung pro ml Ionenaustauscher beaufschlagt. Anschließend wird der Ionenaustauscher zweimal mit Methanol gespült, wobei jeweils 50 % des Säulenvolumens des methanolfeuchten Ionenaustauschers als Spülvolumen eingesetzt wird. Der gesamte Säulenablauf wird aufgefangen und im Wasserstrahlvakuum bei 45°C eingeengt, bis eine ca. 50 Gew.-%ige Lösung von THPE in Methanol erhalten wird. Diese methanolische THPE-Lösung wird unter Rühren bei Raumtemperatur in das doppelte Volumen vollentsalztes Wasser gegeben. Der weiße Niederschlag wird abfiltriert und 24 h bei 50°C sowie 48 h bei 65°C im Wasserstrahlvakuum bis zur Gewichtskonstanz getrocknet.
Ausbeute: 97,5 %
Natriumgehalt 1,1,1-Tris(4-hydroxyphenyl)ethan vor Reinigung: 8,3 ppm
Natriumgehalt 1,1,1-Tris(4-hydroxyphenyl)ethan nach Reinigung: 690 ppb

### Herstellen von verzweigtem Schmelzepolycarbonat mit gereinigtem und nicht gereinigtem 1,1-Tris(4-hydroxyphenyl)ethan (THPE):

### Beispiele 3 und 4

Schmelzepolycarbonat (SPC) wurde in einem mehrstufigen Verfahren hergestellt. Zunächst wurden die Eingangsstoffe BPA, DPC (9000,1 g), Tetraphenylphosphoniumphenolat (0,7 g) und THPE in einem Rührkessel bei ca. 190°C aufgeschmolzen und nach Aufschmelzen 45 min gerührt. Die eingesetzte Menge an BPA wurde so gewählt, dass ein DPC/BPA-Verhältnis auf Basis der Stoffmengen von 110, 108 oder 107 mol-% bezogen auf BPA zu Beginn der Reaktion eingestellt wurde. Der Gehalt an THPE wurde relativ zu BPA auf Basis der Stoffmengen so gewählt, dass 0,3 mol-% bezogen auf BPA an THPE dosiert wurden.

Für das nicht erfindungsgemäße Beispiel 3 wurde kommerziell erhältliches THPE ohne vorherige Aufreinigung verwendet. In dem erfindungsgemäßen Beispiel 4 wurde das gleiche kommerziell erhältliche THPE vor dem Einsatz in der SPC-Reaktion am sauren Ionenaustauscher, wie im Beispiel 1 aufgeführt, aufgereinigt.

Nach einer Reaktionszeit von 45 min bei 190°C bei Atmosphärendruck unter Stickstoff wird die Reaktionsmischung in die Sumpfvorlage eines Fallfilmverdampfers überführt. Im Fallfilmverdampfer wird ausgehend von einer Temperatur von 190°C ein Vakuum von ca. 200 mbar angelegt und das Reaktionsgemisch im Kreislauf über ein von außen beheiztes Fallrohr umgepumpt. Die Umpumpmenge pro Zeit wird über die Versuchszeit konstant gehalten und beträgt bei Beginn der Reaktion im Fallfilmverdampfer das Vierfache des in den Fallfilmverdampfer überführten Flüssigkeitsvolumens pro Stunde. Bei der Reaktion entstehendes Phenol wird abdestilliert, an einem Kondensator auskondensiert und so aus dem Reaktionsgemisch entfernt. Nach einer Verweilzeit von 16 min wird der Druck auf 100 mbar herabgesetzt und die Temperatur auf 220°C erhöht. Das Reaktionsgemisch wird dabei im Kreislauf über das Fallrohr umgepumpt. Nach einer Verweilzeit von 16 min wird der Druck auf 75 mbar herabgesetzt und die Temperatur auf 250°C erhöht. Das Reaktionsgemisch wird dabei im Kreislauf über das Fallrohr umgepumpt. Nach einer Verweilzeit von 16 min wird der Druck auf 50 mbar herabgesetzt und die Temperatur auf 265°C erhöht. Das Reaktionsgemisch wird dabei im Kreislauf über das Fallrohr umgepumpt. Nach einer Verweilzeit von 16 min wird das Reaktionsgemisch in einen Scheibenreaktor überführt. Im Scheibenreaktor kondensiert das Reaktionsgemisch bei drehenden Scheiben und einer Temperatur von 270 - 280°C und einem Druck von 4 - 6 mbar weiter auf, wobei durch die Kondensation gebildetes Phenol kontinuierlich abdestilliert und so aus dem Reaktionsgemisch entfernt wird. Nach 45 min wird der Druck auf 0,5 - 2 mbar reduziert und die Temperatur auf 300 - 310°C erhöht. Das Gemisch wird so lange unter diesen Reaktionsbedingungen gehalten, bis die gewünschte Endviskosität erreicht wird. Anschließend wird die Polymerschmelze aus dem Scheibenreaktor mit Hilfe einer Zahnradpumpe herausgefördert, über eine Düsenplatte ausgetragen und nach Abkühlung und Erstarren in einem Wasserbad anschließend granuliert.

Tabelle 1 gibt eine Übersicht über die Versuche mit gereinigtem und nicht gereinigtem THPE. Beispiel 3 ist nicht erfindungsgemäß, Beispiel 4 ist erfindungsgemäß.
Wie Tabelle 1 zeigt, weist das nicht erfindungsgemäße Beispiel 3 einen deutlich höheren Gehalt der Struktur A von mehr als 600 mg/kg auf, im Gegensatz zu dem erfindungsgemäßen Beispiel 4, in dem der Gehalt der Struktur A kleiner 200 mg/kg ist. Das Verhältnis von THPE zu A ist bei dem nicht erfindungsgemäßen Beispiel deutlich kleiner zehn und bei dem erfindungsgemäßen Beispiel deutlich größer zehn.

**Tabelle 1: Beispiele zur Herstellung von SPC mit unterschiedlichen Mengen und Qualitäten an THPE.**

| Beispiel Nr | Erfindungsgemäß | DPC/ BPA | Mol% THPE | Mw, GPC (UV) [g/mol] | THPE in Produkt [mg/kg] | A in Produkt [mg/kg] | Verhältnis THPE/ A |
|---|---|---|---|---|---|---|---|
| 3 | Nein | 110 | 0,3 | 33474 | 3500 | 644 | 5,4 |
| 4 | Ja | 107 | 0,3 | 25414 | 3600 | 183 | 19,7 |

Bei dem erfindungsgemäßen Beispiel 4 wurde das THPE vor dem Einsatz in der SPC-Reaktion am Ionenaustauscher aufgereinigt.

Das mit gereinigtem THPE hergestellt Schmelzepolycarbonat in Beispiel 4 weist im Vergleich zu Beispiel 3 eine höhere Schmelzeviskosität bei verschiedenen Scherraten aus. Dies ist überraschend, da die relative Viskosität und der Gehalt an THPE (Verzweiger) des nicht erfindungsgemäßen Beispiels 3 vergleichbar mit den Werten des mit erfindungsgemäß gereinigtem THPE hergestellten SPC (Beispiel 4) ist. Das mit gereinigtem THPE hergestellte Schmelzepolycarbonat ist somit deutlich schmelzestabiler als das im nicht erfindungsgemäßen Beispiel 3 mit THPE/A < 8.

## Patentansprüche

1. Tris-hydroxyaryl-Verbindungen mit einem Gehalt an Metall-Ionen von weniger als 10 ppm.

2. Tris-hydroxyaryl-Verbindungen nach Anspruch 1, wobei der Gehalt an Natrium-Ionen weniger als 5 ppm beträgt.

3. Verwendung der Verbindungen nach einem der Ansprüche 1 oder 2 zur Herstellung von verzweigten Polycarbonaten nach dem Umesterungsverfahren mit organischen Carbonaten in der Schmelze.

4. Verwendung eines sauren Ionenaustauschers zur Aufreinigung von Tris-hydroxyaryl-Verbindungen nach Anspruch 1 oder 2.

5. Verfahren zur Herstellung von Tris-hydroxyaryl-Verbindungen nach Anspruch 1 oder 2, **dadurch gekennzeichnet, dass** eine Lösung einer oder mehrerer metallionenhaltiger Tris-hydroxyaryl-Verbindungen mit einem oder mehreren sauren Kationenaustauschern kontaktiert wird.

6. Verfahren zur Aufreinigung von metallionenhaltigen Tris-hydroxyaryl-Verbindungen, umfassend wenigstens folgende Schritte:
a) Konditionieren eines Sulfonsäuregruppen enthaltenden aktiven Ionenaustauschers mit einem Lösemittel, das für die Handhabung der Tris-hydroxyaryl-Verbindungen geeignet ist,
b) Herstellen einer Lösung der zu reinigenden Tris-hydroxyaryl-Verbindungen in einem Lösemittel, das für die Handhabung der Tris-hydroxyaryl-Verbindungen geeignet ist,
c) kontinuierliches oder diskontinuierliches Kontaktieren der Tris-hydroxyaryl-Verbindung-haltigen Lösung aus b) mit einem konditionierten Ionenaustauscher aus a),
d) Abtrennen der Tris-hydroxyaryl-Verbindung-haltigen Lösung aus c) von dem konditionierten Ionenaustauscher,
e) wenigstens teilweises Entfernen des Lösemittels aus der in c) gereinigten Lösung der Tris-hydroxyaryl-Verbindung unter geringer Temperatur-Belastung,

7. Verfahren nach Anspruch 6, **dadurch gekennzeichnet, dass**, unter der Maßgabe, dass das Lösemittel in Schritt e) nur teilweise entfernt wurde, anschließendes Fällen der Tris-hydroxyaryl-Verbindung in einem geeigneten Fällungsmittel und schonendes Trocknen unter geringer Temperatur-Belastung erfolgt.

8. Verfahren nach Anspruch 6 oder 7, **dadurch gekennzeichnet, dass** das Entfernen des Lösemittels in Schritt e) und / oder die Trocknung der Tris-hydroxyaryl-Verbindungen unter Vakuum erfolgt.

9. Verfahren nach einem der Ansprüche 5 bis 8, **dadurch gekennzeichnet, dass** der Ionenaustauscher vor der Konditionierung mit Wasser gewaschen wird, bis die Leitfähigkeit im Ablauf der Säule weniger als 50 µS/cm, bevorzugt weniger als 20 µS/cm und anschließend mit dem Lösemittel, das für die Handhabung der Tris-hydroxyaryl-Verbindungen geeignet ist, so lange gespült wird, bis der Wassergehalt im lösemittelhaltigen Ablauf weniger als 2 Gew.-% aufweist.

10. Verfahren nach Anspruch 4 oder 5, **dadurch gekennzeichnet, dass** der Kontakt der Tris-hydroxyaryl-Verbindungen in Lösung mit den Ionenaustauschern bei Temperaturen von -50 bis 120°C durchgeführt wird.

11. Verfahren nach einem der Ansprüche 4 bis 6, **dadurch gekennzeichnet, dass** der Kontakt der Tris-hydroxyaryl-Verbindungen mit den Ionenaustauschern batch-weise mindestens einmal oder in einem kontinuierlichen Verfahren vorgenommen wird.

12. Verfahren nach Anspruch 11, **dadurch gekennzeichnet, dass** im kontinuierlichen Verfahren in Schritt c) eine Schüttung von Ionenaustauschern in Form von sogenannten Festbetten verwendet wird.

13. Verfahren nach einem der Ansprüche 4 bis 7, **dadurch gekennzeichnet, dass** die Tris-hydroxyaryl-Verbindung 1,1,1-Tris(4-hydroxyphenyl)ethan und das verwendete Lösemittel Methanol oder Phenol ist.

14. Verfahren nach einem der Ansprüche 4 bis 7, **dadurch gekennzeichnet, dass** saure Ionenaustauscher auf Basis vernetzter oder teilvernetzter Polystyrole eingesetzt werden.

15. Verfahren nach einem der Ansprüche 4 bis 7, **dadurch gekennzeichnet, dass** saure Ionenaustauscher auf Basis vernetzter oder teilvernetzter Polyacrylate eingesetzt werden.

16. Verfahren nach einem der Ansprüche 4 bis 7, **dadurch gekennzeichnet, dass** saure Ionenaustauscher funktionalisiert mit einer oder mehreren Funktionen aus der Gruppe Sulfonsäure, Carbonsäure, Phosphonsäure, Perchlorsäure verwendet werden.
